# EUROPEAN PATENT APPLICATION

(11) **EP 1 528 500 A1**
(43) Date of publication of application: **04.05.2005**
(21) Application number: 03078401.1
(22) Date of filing: 28.10.2003
(51) Int. Cl.: G06F 19/00

(54) **Electronic prescription system and method**

(71) Applicant: Chhabra International Ltd, Dublin (IE)
(72) Inventor: Keenen, Ian, Dublin 2 (IE)
(74) Representative: van Westenbrugge, Andries

(57) **Abstract**

Electronic prescription system and method, the system comprising a customer server (16) for providing access to the electronic prescription system (1) by a customer, a prescription server (15) which is connectable to the customer server (16) for processing a prescription order, and supply server (18) for managing shipment of products associated with the prescription order on basis of input data received from the prescription server (15). The prescription server (15) is connectable to a database (17) comprising prescription rules. The prescription server (15) is arranged to send the prescription order to the supply server (18) only after checking the prescription order and the input data against the prescription rules. The shipment of the order is being tracked, and the prescription order is being billed when the shipment has actually arrived.

## Description

### Field of the invention

The present invention relates to an electronic prescription system and method for providing products associated with a prescription order, such as drugs or medicine, to a person. More particularly, the present invention relates to an electronic prescription system comprising a customer server for providing access to the electronic prescription system by a customer, a prescription server which is connectable to the customer server for processing a prescription order, and a supply server for managing shipment of products associated with the prescription order on basis of input data received from the prescription server.

### Prior art

Such an electronic prescription system is known from international patent application WO01/77927. This publication discloses an electronic prescription system, in which a prescription is electronically delivered from the prescribing physician to a specified pharmacy. The pharmacy may be specified by the patient or may be determined automatically as the pharmacy nearest to the patient's residence. The electronic prescription system stores all issued prescriptions in a central database to allow using the medical information for (national) statistical purposes. Also, a patient database is maintained.

The electronic prescription system according to WO01/77927 only provides for electronic routing of a prescription in electronic form in the usual way: a patient visits a physician for a medical consult, the physician issues a prescription, and a pharmacy provides the medicine according to the prescription to the patient. In addition a patient database is maintained, as well as a central database. This electronic prescription system thus still has a number of disadvantages which are also associated with the normal way of issuing a prescription.

### Summary of the invention

The present invention seeks to provide an electronic prescription system and method, which allows improvements over prior art electronic and conventional prescription systems.

According to a first aspect of the present invention, an electronic prescription system according to the preamble defined above is provided, in which the prescription server is connectable to a database comprising prescription rules, and in which the prescription server is arranged to send the prescription order to the supply server only after checking the prescription order and the input data against the prescription rules. This allows an automatic checking of the prescription order before shipment of the products associated with the prescription order. In this case, the customer is a physician in e.g. a hospital or clinic, which issues a prescription order to the person, e.g. after a consultation.

The prescription rules stored in the database may comprise general rules and personal rules, in which the personal rules are associated with a person for which the prescription order is issued. This allows checking of a prescription order against general rules, such as checks against simultaneous use of other drugs which may be dangerous when used in combination, and against personal rules, such as a sensitivity against a specific family of drugs. Also, the prescription rules may comprise a check against previous medication of a same type, a check against use of other medications, and/or a check against projected or predicted usage of medication. In this manner, safeguards can be built into the system which will allow to issue a warning when abnormal situations arise (sudden increase of ordering of a specific drug).

In a further embodiment, the supply server is arranged to track a shipment of products according to the prescription order. The tracking information can then be made available to the person expecting reception of the products associated with the prescription order.

The electronic prescription system may further comprise a billing server connected to the prescription server for executing a billing process once the shipment has actually been delivered. By only starting the billing process after actual delivery of the products (or actual reception by the person), it is possible to minimize the number of false billing procedures. As correction of a false billing procedure is time consuming and costly, this embodiment provides economical advantages.

In an even further embodiment, the prescription server is further arranged to update and store product catalogues for each of the customers based on input received from the supply server. The physician can then select from an up to date catalogue and benefit from the availability of new products or interesting offers.

In the above embodiment, the physician provides the prescription order and inputs it into the electronic prescription system. In a further series of embodiments, it is possible for a person seeking a prescription for a certain ailment or disease, to obtain a prescription without actually having to visit a physician. In this embodiment, the database comprises questionnaire rules, and the prescription server is further arranged for receiving questionnaire data from a person and for assembling a prescription order based on the questionnaire data and the questionnaire rules. The prescription order can then be processed by the electronic prescription system as described above. This embodiment is particularly suited for obtaining so called over-the-counter medicine, which are available without actual consultation of a physician.

In an even further embodiment, the customer server or prescription server is further arranged for receiving questionnaire data from a person, sending the questionnaire data to a customer client connected to the customer server for review by the customer, and providing a prescription order based on the questionnaire data and customer review. In this embodiment, a physician reviews the questionnaire data and or the (automatically generated) prescription order before the prescription order is further processed. This provides an additional safeguard for delivery of over-the-counter medicine, or allows to even deliver prescription medicine to a person without the person having to physically visit a physician.

According to a further aspect of the present invention, an electronic prescription method is provided for providing products associated with a prescription order for a person, comprising providing access to a customer, processing the prescription order, and shipping the products associated with the prescription order on basis of input data received from the customer only after checking the prescription order and the input data against predefined prescription rules. This method and further method embodiments as described by the dependent method claims provide substantially the same advantages as the electronic prescription system according to the present invention.

An even further aspect according to the present invention relates to a computer program product comprising computer executable code, which after loading the computer executable code in a computer system comprising one or more interconnected processing systems, provides the computer system with the ability to perform the present electronic prescription method.

### Short description of drawings

The present invention will be discussed in more detail below, using a number of exemplary embodiments, with reference to the attached drawings, in which
Fig. 1 shows a diagrammatic view of an electronic prescription system according to a first embodiment of the present invention; and
Fig. 2 shows a flow diagram associated with an embodiment of the present invention.

### Detailed description of exemplary embodiments

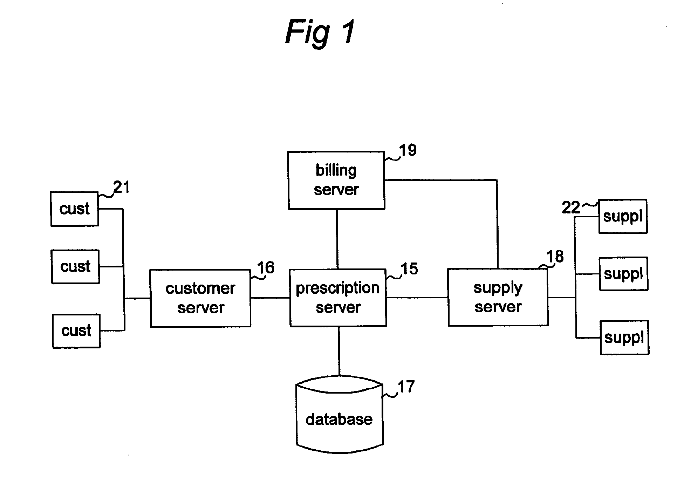

In Fig. 1, a schematic diagram is shown of a first embodiment of the electronic prescription system 1 according to the present invention. In the diagram, a number of servers are shown. These servers may be implemented on general purpose or dedicated computers or processing systems. One or more servers may also be combined to be implemented or executed on a single computer.

The central node in the diagram is the prescription server 15, which is arranged to control the complete electronic prescription system 1. The prescription server 15 is connected to a customer server 16, which is arranged to provide an interface to users of the system. The customer server 16 may e.g. be a website server, which is able to communicate with a number of customer clients 21 via the world wide web. The customer clients 21 may e.g. be computers connected to the internet at a site of a physician (e.g. a hospital or a clinic).

Furthermore, the prescription server 15 is connected to a database 17, which is arranged to store data related to the electronic prescription system 1, the specific details of which will be discussed below. The data may be stored temporarily, or indefinitely. The other servers in the electronic prescription system 1 may use this single database 17, or may be provided with separate data storage means (local semiconductor memory or other local storage, such as magnetic or optical disc storage).

The drugs or medicine associated with the prescriptions originate from a manufacturer or wholesaler. These entities are in contact with the prescription server 15 by means of a supply server 18. As illustrated in Fig. 1, a number of supply clients 22 (again, e.g. implemented on a general purpose computer system) are connectable to the supply server 18, e.g. via the Internet or another data network. The supply server 18 is arranged to route prescription orders and associated data and to update and maintain product catalogues of the manufacturers and wholesalers.

Also, a billing server 19 is connected to the prescription server 15 (and possibly to the supply server 18). The billing server is arranged to control and monitor the billing process of the present electronic prescription system 10.

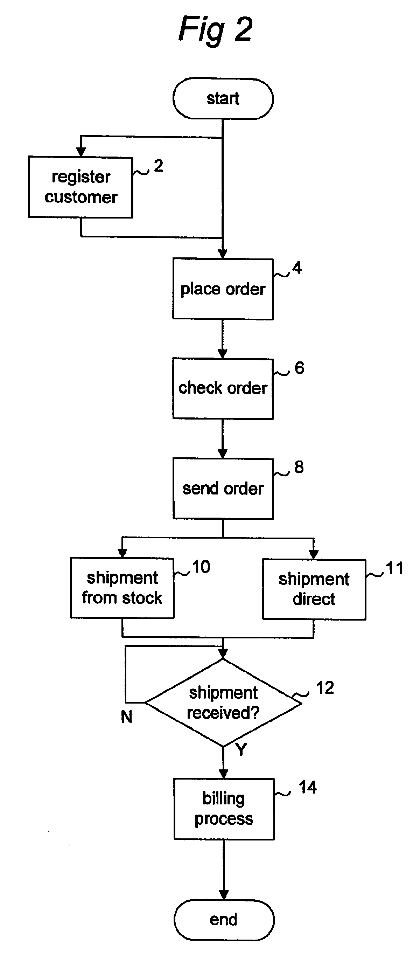

In Fig. 2, a flow chart is shown relating to a first embodiment according to the present invention, in which a treating physician (in a hospital, a clinic, or a private doctor) uses the prescription system 1 to provide a medication to a visiting patient.

In a first step 2, all parties (the doctor who issues a prescription, and the possible medicine suppliers, i.e. manufacturer or wholesaler) must register and the registration process must be validated. Data from a completed questionnaire is used to set up a personal customer account with restricted access, which may e.g. be stored in the database 17. In this first embodiment, the customer is understood to be a physician from a hospital or clinic, or a private physician, such as a general practitioner or a pharmacist. Using real data from patient databases provided by a third party **[WHO?? Ian, please elaborate],** algorithms are constructed to determine the parameters of drug usage by department.

In a next step 4, an order for a prescription is placed by the customer, using the customer client 21 and customer server 16 as described above. The customer can view products in its customer specific catalogue, which is stored in the database 17. The customer can accept the offered price or request a quote. The quote is presented by the system 1 using the customer server 16, and the customer can accept or decline the quote.

In a following step, the orders placed are cross checked and validated against a number of possible criteria (step 6, using the prescription server 15), e.g. credit history, previous order quantities and/or a drug specific algorithm. The drug specific algorithm is e.g. a check against predicted or projected usage in a hospital. If e.g., a hospital has a renal medicine department, it is likely to have a need for erythropoetin (a drug used for patients undergoing dialysis). The number of treated patients and the period of treatment, multiplied by the dosage of the drug used, will determine the quantity of drug required in a predetermined period.

Also, the prescription order may be checked using general rules and personal medicine rules. The prescription order is e.g. checked against prior use by the associated patient (e.g. quantity), use of other medicine by the associated patient (to prevent possible harmful combinations of medicine).

In addition, the specific shipment documentation may be selected, comprising one or more from the following documentation: pro-forma invoice, number of invoices, airway bill, certificate of analysis (document that states that the drug to be imported has been analysed and conforms to the registration in place for that drug in the country of import).

Furthermore, the payment method required by the manufacturer or wholesaler may be selected, e.g. a letter of credit, an irrevocable letter of credit, a BACS transfer or other credit terms accepted, and associated information is sent to the billing server 19.

In a following step 8, the prescription order is routed internally in the electronic prescription system 1 when the drug specified is in stock. A manufacturer or wholesaler can require that the organisation executing the present electronic prescription system maintains a buffer stock for certain specified drugs. Alternatively the prescription order is sent directly to either a manufacturer of the specific drug associated with the prescription, or to a wholesaler of the specific drug, using the supply server 18. If the prescription order is sent internally, then an inventory management process (e.g. implemented in the supply server 18) is notified to contact the manufacturer and reorder replacement stock as necessary.

If the prescription order is sent to the manufacturer, then a shipment status must be notified to the supply server 18. The prescription server 15 may forward the shipping information, e.g. a tracking number, to the customer server 21, to allow on-line tracking of the order. Additionally, a shipping provider who ships the drugs from the stock to the customer, may be linked via EDI to the prescription server 15 so that the customer can track the prescription order.

The billing process is executed on the billing server 19. The billing process is managed by the electronic prescription system 1, regardless of whether the customer's order is placed on the manufacturer, the wholesaler or on the system's stock. The billing server 19 is arranged to receive a payment, e.g. into an escrow account, to debit any commission and to pay the supplier. When using credit card payments, there may be problems arising when a shipment is debited without the shipment actually reaching the customer. To minimise this amount of false payments (which have to be reversed at a considerable cost), the billing server 19 only starts the billing process when notice is received of the actual reception of the ordered prescription drug by the customer. This can be implemented using the shipping provider's information (actual receipt).

The prescription server 15 is also arranged to maintain a plurality of customer catalogues, and store these in the database 17. Each customer has a personal catalogue that can be added to at the discretion of individual manufacturers or wholesalers. Once a manufacture has added a product, the same product from a wholesaler is blocked so that orders are directed to the manufacturer or the present electronic prescription system 1 for that product. Manufacturers or wholesalers can upload prices at any time for any customer using the supply server 18 and prescription server 15. Special offers can be displayed on the customer's web page or e-mailed directly to them.

In some countries, it is possible to have a patient order the prescription after receiving the prescription (in paper or electronic from) from a treating physician or doctor. In this case, the customer server 16 is contacted by the patient. In addition to the data as described above, the prescription server is then also arranged to check the patient contact data, and the database 17 also comprises patient data. E.g., it can then be checked whether the patient is already registered (and all patient data, including insurance data, known). If not, the prescription server 15 will receive the relevant data from the patient, e.g. using predefined web forms, and check for insurance eligibility and shipment data. When the patient is already known, or is being approved in the above described process, the prescription order is processed in the normal way (transfer of prescription order 8, shipment of prescription product(s) 10, 11, tracking 12 and billing 14, see Fig. 2).

The prescription server 15 is arranged to notify the patient, e.g. by e-mail, in any possible exception case (e.g. when the patient is not eligible for insurance coverage for that particular prescription).

A patient or person having a need for a certain medicine or drug, may use an electronic prescription system according to a second embodiment of the present invention. In this embodiment, the person contacts the electronic prescription system 1, e.g. using a web based access (visiting an Internet page). This may be implemented in the customer server 16 of Fig. 1. Here, the person can enter data in a questionnaire relating to general medical questions and specific medical questions (e.g. specific for a certain kind of medical condition).

Additionally, the questionnaire data are sent to a customer client 22 via the customer server 16 for review by a (licensed) physician and provision of a prescription order. The review by the physician may result in a cancellation of the prescription order, the prescription order may be (partially) declined by the physician (after which a reasoned statement is sent to the patient), or the prescription order may be accepted. After acceptation of the prescription order, it is resent to the prescription server 15, which then follows an identical process as described above in relation to the first embodiment.

In an alternative embodiment, the questionnaire data is gathered by the prescription server 15 and checked against questionnaire rules in the database 17. In the database 17, questionnaire rules may be present which are used by the prescription server 15 to assemble a prescription order (type and dosage of medicine or drug). This prescription order is then checked against the general and specific prescription rules in the database 17 (checks for contra-indicators and other safety checks), as in the first embodiment described above. Also, the prescription order may be sent to a physician for review as described in the paragraph above.

The questionnaire comprises a number of general questions, such as date of birth, height, weight, sex. Furthermore, the questionnaire comprises general medical questions, such as listed in table I below. Also, the questionnaire may comprise further information questions and disclaimers. The questionnaire may also comprise questions, which are specific for a certain medical condition, such as obesity.

As the data which is being interchanged in the present electronic prescription system 1 is, generally speaking, of a confidential nature, the transmission of this data is performed by scrambling the data or by using secure communication channels.

**Table I**

| **Questionnaire** | |
|---|---|
| Blood pressure (ca 120/80 mm Hg = normal) | normal/high/low |
| Do you smoke? | yes/no |
| Do you consume alcoholic beverages? | no/yes (more than 2 glasses a day) |
| Are you allergic to any medication or substance? | Yes/no |
| Are you currently taking any medicines? | Yes/no |
| Do you have any disease, disorder or medical problem? | No/yes (specify) |
| Did you have serious surgery? | Yes/no |
| Is there anything else the prescribing doctor needs to know? | No/yes (specify) |
| If the medical doctor needs more information he may contact me on my e-mail address. | Yes/no |

| **Disclaimers** | |
|---|---|
| I agree to read the patient information leaflet before taking the medication. | Yes/no |
| I agree to stop taking my medication, if I am or become allergic to the medication. | Yes/no |
| I agree not to take this medicine if I have any pre-existing skin disorders. | Yes/no |
| I agree to limit sun exposure and to avoid sunlamps while on this medication. | Yes/no |
| I agree not to take any over-the-counter medicines without approval from my pharmacist. | Yes/no |
| I agree not to take this medication if I am pregnant, breast-feeding, or trying to get pregnant. | Yes/no |

## Claims

1. Electronic prescription system (1) comprising:
- a customer server (16) for providing access to the electronic prescription system (1) by a customer,
- a prescription server (15) which is connectable to the customer server (16) for processing a prescription order, and
- a supply server (18) for managing shipment of products associated with the prescription order on basis of input data received from the prescription server (15),
in which the prescription server (15) is connectable to a database (17) comprising prescription rules, and in which the prescription server (15) is arranged to send the prescription order to the supply server (18) only after checking the prescription order and the input data against the prescription rules.

2. Electronic prescription system according to claim 1, in which the prescription rules stored in the database (17) comprise general rules and personal rules, in which the personal rules are associated with a person for which the prescription order is issued.

3. Electronic prescription system according to claim 1 or 2, in which the prescription rules comprise a check against previous medication of a same type, a check against use of other medications, and/or a check against projected or predicted usage of medication.

4. Electronic prescription system according to one of the proceeding claims, in which the supply server (18) is arranged to track a shipment of products according to the prescription order.

5. Electronic prescription system according to claim 4, in which the electronic prescription system further comprises a billing server (19) connected to the prescription server (15) for executing a billing process once the shipment has actually been delivered.

6. Electronic prescription system according to one of the proceeding claims, in which the prescription server (15) is further arranged to update and store product catalogues for each of the customers based on input received from the supply server (18).

7. Electronic prescription system according to one of the proceeding claims, in which the database (17) comprises questionnaire rules, and the prescription server (15) is further arranged for receiving questionnaire data from a person and for assembling a prescription order based on the questionnaire data and the questionnaire rules.

8. Electronic prescription system according to one of the claims 1 through 6, in which the customer server (16) or prescription server (15) is further arranged for
- receiving questionnaire data from a person,
- sending the questionnaire data to a customer client (2 1 ) connected to the customer server (16) for review by the customer;
- providing a prescription order based on the questionnaire data and customer review.

9. Electronic prescription method for providing products associated with a prescription order for a person, comprising:
- providing access to a customer,
- processing the prescription order, and
- shipping the products associated with the prescription order on basis of input data received from the customer only after checking the prescription order and the input data against predefined prescription rules.

10. Electronic prescription method according to claim 9, in which the prescription rules comprise general rules and personal rules, in which the personal rules are associated with the person for which the prescription is issued.

11. Electronic prescription method according to claim 9 or 10, in which the prescription rules comprise a check against previous medication of a same type, a check against use of other medications, and/or a check against projected or predicted usage of medication.

12. Electronic prescription method according to one of the claims 9 through 11, in which products according to the prescription are being tracked.

13. Electronic prescription method according to claim 12, in which a billing process is executed once the shipment has actually been delivered.

14. Electronic prescription method according to one of the claims 9 through 13, in which product catalogues for each of the customers are updated and stored based on input received from a supplier.

15. Electronic prescription method according to one of the claims 9 through 15, in which questionnaire data is received from a person and a prescription order is assembled based on the questionnaire data and questionnaire rules.

16. Electronic prescription method according to one of the claims 9 through 15, in which
- questionnaire data are received from a person,
- the questionnaire data are sent to the customer for review by the customer; and
- a prescription order is provided based on the questionnaire data and customer review.

17. Computer program product comprising computer executable code, which after loading the computer executable code in a computer system comprising one or more interconnected processing systems, provides the computer system with the ability to perform the electronic prescription method according to one of the claims 9 through 16.
